# EUROPEAN PATENT SPECIFICATION

(11) **EP 3 004 302 B1**
(45) Date of publication and mention of the grant of the patent: **04.01.2017**
(21) Application number: 14724403.2
(22) Date of filing: 12.05.2014
(51) Int. Cl.: C11B 9/00, C07C 49/553, A61Q 13/00, A61L 9/01, C11D 3/50

(54) **ODORANT KETONES**
KETON-DUFTSTOFFE
CÉTONES ODORANTES

(30) Priority: 27.05.2013 EP 13169301
(43) Date of publication of application: 13.04.2016
(73) Proprietor: Firmenich SA, 1211 Geneva 8 (CH)
(72) Inventor: BIRKBECK, Anthony A., CH-1211 Geneva 8 (CH)
(74) Representative: Carina, Riccardo Filippo
(86) International application number: PCT/EP2014/059664
(87) International publication number: WO 2014/191187

(56) References cited:
- EP-A1- 0 743 297
- EP-A2- 0 464 357

## Description

### Technical field

The present invention relates to the field of perfumery. More particularly, it concerns compounds of formula (I), as defined below, which are useful perfuming ingredients of the woody, balsamic type. The present invention concerns also the invention's compounds as part of a perfuming composition or of a perfuming consumer product.

### Prior art

To the best of our knowledge, the invention's compounds of formula (I) are novel.

To the best of our knowledge, the closest analogues known in the perfumery are the ones described in US 3929677 or in EP464357. However, the invention's compounds differ from the latter by having a significantly different chemical structure and its specific odor profile. This prior art document does not report or suggest any organoleptic properties of the compounds of formula (I) and does not report or suggest any use of said compounds in the field of perfumery.

### Description of the invention

We have now surprisingly discovered that a compound of formula 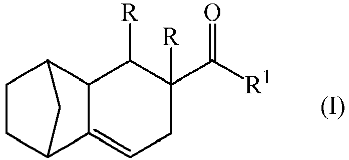 in the form of any one of its stereoisomers or a mixture thereof, and wherein each R, independently from each other, represents a hydrogen atom or a methyl or ethyl group provided that at least one of said R groups represents a methyl or ethyl group; and R¹ represents a methyl or ethyl group;
can be used as perfuming ingredient, for instance to impart odor notes of the woody, balsamic type.

The odor of the family is here described as including, inter alia, woody notes but it is intended that the various compounds do have various shades of the woody family. These shades may include camphor, earthy, pine and/or cedar aspect.

According to a particular embodiment of the invention, said compound (I) can be of formula 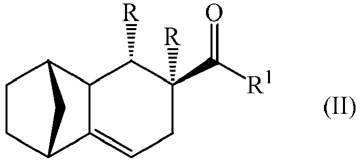 wherein R and R¹ have the same meaning as above, and the bold and hatched lines represent the relative stereochemistry of the various groups.

For the sake of clarity, by the expression "any one of its stereoisomers", or the similar, it is meant the normal meaning understood by a person skilled in the art, i.e. that the invention's compound can be a pure enantiomer (if chiral) or diastereomer (such as the endo/exo isomers). According to any one of the above embodiments of the invention, said compound is in the form of a mixture of diastereomers wherein the bridging group and the ketone group are in a relative configuration endo or exo and the endo diastereomer accounts for at least 60% w/w of the mixture.

According to any one of the above embodiments of the invention, at least one of said R groups represents a methyl or ethyl group and at least one of said R groups represents a hydrogen atom or a methyl group.

According to any one of the above embodiments of the invention, the two R groups represent each a methyl group.

According to any one of the above embodiments of the invention, R¹ represents a methyl group.

According to any one of the above embodiments of the invention, said compounds (I) are C₁₄-C₁₅ compounds.

The compounds of formula (I) are new compounds, and therefore also an object of the present invention.

As specific examples of the invention's compounds, one may cite, as non-limiting example, 1-((1RS,4SR,6RS)-5,6-dimethyl-1,2,3,4,4a,5,6,7-octahydro-1,4-methanonaphthalen-6-yl)ethanone which is a preferred embodiment of the invention and possesses an odor characterized by an elegant balsamic-tonka note and a woody- cedar, camphor earthy, pine note as well as by having an allylic-pineapple shade.

As other example, one may cite 1-((1RS,4SR,6RS)-6-methyl-1,2,3,4,4a,5,6,7-octahydro-1,4-methanonaphthalen-6-yl)ethanone, which possesses a woody-camphor, earthy notes, with a balsamic and aromatic bottom note.

When the odor of the invention's compounds is compared with that of the prior art compounds mentioned above, then the invention's compounds distinguish themselves by lacking, or not possessing a significant, flowery and/or ambery notes which are characteristic of the prior art compounds, and differs also by having a balsamic note. Said differences lend the invention's compounds and the prior art compounds to be each suitable for different uses, i.e. to impart different organoleptic impressions.

As mentioned above, the invention concerns the use of a compound of formula (I) as perfuming ingredient. In other words, it concerns a method to confer, enhance, improve or modify the odor properties of a perfuming composition or of a perfumed article, which method comprises adding to said composition or article an effective amount of at least a compound of formula (I). By "use of a compound of formula (I)" it has to be understood here also the use of any composition containing a compound (I) and which can be advantageously employed in perfumery industry.

Said compositions, which in fact can be advantageously employed as perfuming ingredients, are also an object of the present invention.

Therefore, another object of the present invention is a perfuming composition comprising:
i) as perfuming ingredient, at least one invention's compound as defined above;
ii) at least one ingredient selected from the group consisting of a perfumery carrier and a perfumery base; and
iii) optionally at least one perfumery adjuvant.

By "perfumery carrier" we mean here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the most commonly used. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company).

As solid carriers one may cite, as non-limiting examples, absorbing gums or polymers, or yet encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloide: Stabilisatoren, Dickungs- und Geliermittel in Lebensmitteln, Band 2 der Schriftenreihe Lebensmittelchemie, Lebensmittelqualitat, Behr's Verlag GmbH & Co., Hamburg, 1996. The encapsulation is a well known process to a person skilled in the art, and may be performed, for instance, using techniques such as spray-drying, agglomeration or yet extrusion; or consists of a coating encapsulation, including coacervation and complex coacervation technique.

By "perfumery base" we mean here a composition comprising at least one perfuming co-ingredient.

Said perfuming co-ingredient is not of formula (I). Moreover, by "perfuming co-ingredient" it is meant here a compound, which is used in a perfuming preparation or a composition to impart a hedonic effect. In other words such a co-ingredient, to be considered as being a perfuming one, must be recognized by a person skilled in the art as being able to impart or modify in a positive or pleasant way the odor of a composition, and not just as having an odor.

The nature and type of the perfuming co-ingredients present in the base do not warrant a more detailed description here, which in any case would not be exhaustive, the skilled person being able to select them on the basis of his general knowledge and according to intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

An invention's composition consisting of at least one compound of formula (I) and at least one perfumery carrier represents a particular embodiment of the invention as well as a perfuming composition comprising at least one compound of formula (I), at least one perfumery carrier, at least one perfumery base, and optionally at least one perfumery adjuvant.

It is useful to mention here that the possibility to have, in the compositions mentioned above, more than one compound of formula (I) is important as it enables the perfumer to prepare accords, perfumes, possessing the odor tonality of various compounds of the invention, creating thus new tools for his work.

For the sake of clarity, it is also understood that any mixture resulting directly from a chemical synthesis, e.g. a reaction medium without an adequate purification, in which the compound of the invention would be involved as a starting, intermediate or end-product could not be considered as a perfuming composition according to the invention as far as said mixture does not provide the inventive compound in a suitable form for perfumery. Thus, unpurified reaction mixtures are generally excluded from the present invention unless otherwise specified.

Furthermore, the invention's compound can also be advantageously used in all the fields of modern perfumery, i.e. fine or functional perfumery, to positively impart or modify the odor of a consumer product into which said compound (I) is added. Consequently, another object of the present invention is represented by a perfuming consumer product comprising, as perfuming ingredient, at least one compound of formula (I), as defined above.

The invention's compound can be added as such or as part of an invention's perfuming composition.

For the sake of clarity, it has to be mentioned that, by "perfuming consumer product" it is meant a consumer product which is expected to deliver at least a pleasant perfuming effect to the surface to which it is applied (e.g. skin, hair, textile, or home surface). In other words, a perfuming consumer product according to the invention is a perfumed consumer product which comprises the functional formulation, as well as optionally additional benefit agents, corresponding to the desired consumer product, e.g. a detergent or an air freshener, and an olfactive effective amount of at least one invention's compound.

The nature and type of the constituents of the perfumery consumer product do not warrant a more detailed description here, which in any case would not be exhaustive, the skilled person being able to select them on the basis of his general knowledge and according to the nature and the desired effect of said product.

Non-limiting examples of suitable perfumery consumer product can be a perfume, such as a fine perfume, a cologne or an after-shave lotion; a fabric care product, such as a liquid or solid detergent, a fabric softener, a fabric refresher, an ironing water, a paper, or a bleach; a body-care product, such as a hair care product (e.g. a shampoo, a coloring preparation or a hair spray), a cosmetic preparation (e.g. a vanishing cream or a deodorant or antiperspirant), or a skin-care product (e.g. a perfumed soap, shower or bath mousse, oil or gel, or a hygiene product); an air care product, such as an air freshener or a "ready to use" powdered air freshener; or a home care product, such as a wipe, a dish detergent or hard-surface detergent.

Some of the above-mentioned consumer products may represent an aggressive medium for the invention's compound, so that it may be necessary to protect the latter from premature decomposition, for example by encapsulation or by chemically bounding it to another chemical which is suitable to release the invention's ingredient upon a suitable external stimulus, such as an enzyme, light, heat or a change of pH.

The proportions in which the compounds according to the invention can be incorporated into the various aforementioned articles or compositions vary within a wide range of values. These values are dependent on the nature of the article to be perfumed and on the desired organoleptic effect as well as the nature of the co-ingredients in a given base when the compounds according to the invention are mixed with perfuming co-ingredients, solvents or additives commonly used in the art.

For example, in the case of perfuming compositions, typical concentrations are in the order of 0.01 % to 60 % by weight, or even more, of the compounds of the invention based on the weight of the composition into which they are incorporated. Concentrations lower than these, such as in the order of 0.001% to 20% by weight, can be used when these compounds are incorporated into perfumed articles, percentage being relative to the weight of the article.

The invention's compounds can be prepared from 2-norbornene according to a method as described in Scheme 1:

### Scheme 1: General synthetic pathway

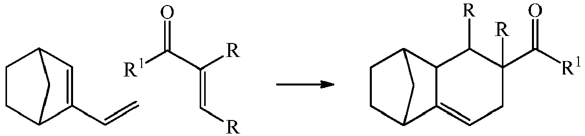

wherein R and R¹ have the meaning indicated above. The Examples herein below show different examples of such approach.

### Examples

The invention will now be described in further detail by way of the following examples, wherein the abbreviations have the usual meaning in the art, the temperatures are indicated in degrees centigrade (°C) ; the NMR spectral data were recorded in CDCl₃ (if not stated otherwise) with a 360 or 400 MHz machine for ¹H and ¹³C, the chemical shifts δ are indicated in ppm with respect to TMS as standard, the coupling constants J are expressed in Hz.

### Example 1

### Synthesis of compounds of formula (I)

### • Preparation of the starting diene :

### (1RS, 4RS)-2-ethynylbicyclo[2.2.1]heptan-2-ol

To a solution of ethynyl magnesium bromide (0.5 M in THF, 900 mL) cooled to 0-5°C with an ice bath was added slowly dropwise a stirred solution of norcamphor (40 g, 363 mmol) in THF (500 mL). The solution was allowed to warm slowly to ambient temperature and stirred for further 24 hrs, then poured into ice water. Extracted with ether, washed with saturated ammonium chloride then brine, dried over sodium sulfate, filtered and the solvents removed *in vacuo* to yield the crude propargylic alcohol. Bulb to bulb distillation 0.05 mbar at 35-40°C gave the pure alcohol, 36.2 g (yield: 73%).
¹³C NMR: 89.8 (q), 73.3 (q), 71.0, 49.7 (CH), 47.7, 38.8 (CH₂), 36.9 (CH), 28.7, 21.0 (CH₂)

### (1RS,4RS)-2-vinylbicyclo[2.2.1]heptan-2-ol

Lindlar catalyst (1.0g, 5% Pd/C on BaSO₄ Fluka 62145) was added to a solution of the alcohol obtained above (42 g, 294 mmol) in cyclohexane (400 mL) then evacuated in vacuo and purged with hydrogen gas three times then shaken under an atmosphere of hydrogen gas (1 atm) for 3 hours. The suspension was then filtered and the solvents removed *in vacuo* to yield the allylic alcohol.
¹³C NMR: 145.6 (q), 110.3 (CH₂,), 79.2 (q), 47.4 (CH), 45.0, 38.3 (CH₂), 37.2 (CH), 28.9, 21.9 (CH₂)

### (1RS, 4RS)-2-vinylbicyclo[2.2.1]heptan-2-ene

Amberlyst 15 (4.0 g) was added to a solution of the allylic alcohol obtained above (21 g, 152 mmol) in pentane (300 mL) and the suspension heated at 35-40°C for 5-7 hours, then cooled and filtered. This solution was used directly in the next step without further purification.
¹³C NMR: 148.1 (q), 133.9, 132.0 (CH), 112.3 (CH), 47.6 (CH₂), 42.7, 41.0 (CH), 27.4, 24.7 (CH₂)

### • Preparation of compounds of formula (I):

### 1-((1RS,4SR,6RS)-6-methyl-1,2,3,4,4a,5,6,7-octahydro-1,4-methanonaphthalen-6-yl)ethanone

To a stirred solution of 3-methylbut-3-en-2-one (0.84 g, 10 mmol) in dichloromethane (25 mL) cooled to -40°C (dry ice ethanol bath) was added slowly dropwise a solution of ethyl aluminium dichloride (9 mL, 1.0 M in hexanes, 9 mmol). After 5 minutes, a solution of (1RS, 4RS)-2-vinylbicyclo[2.2.1]heptan-2-ene (1 g, 8.32 mmol) in pentane (15 mL) was added slowly dropwise. The suspension was allowed to slowly warm to ambient temperature and then stirred for a further 12 hours, then poured into ice/water and stirred for 30 minutes, then extracted with ether, washed organic phase with saturated ammonium chloride, then brine, dried over sodium sulfate, filtered and the solvents removed in vacuo to yield the crude ketone, which was further purified by bulb to bulb distillation, 0.05 mbar, 130-140 °C gave the desired ketone, 180 mg.
¹³C NMR: 213.4 (q), 150.9 (q), 111.0 (CH), 46.9 (q), 44.1, 40.2, 40.0 (CH), 37.7, 35.4, 33.0, 30.9, 26.6 (CH₂), 24.8, 23.9 (CH₃)

### 1-5,6-dimethyl-1,2,3,4,4a,5,6,7-octahydro-1,4-methanonaphthalen-6-yl)ethanone; and 1-((1RS,4SR,6RS)-5,6-dimethyl-1,2,3,4,4a,5,6,7-octahydro-1,4-methanonaphthalen-6-yl)ethanone

A solution of ethyl aluminum dichloride (75 mL, 1.0 M in hexanes, 75 mmol) was added slowly dropwise to a stirred solution of (E)-3-methylpent-3-en-2-one (16.2 g, 165 mmol) in dichloromethane (200 mL) cooled to -40°C (dry ice ethanol bath). After 15 minutes at -40°C, a solution of (1RS, 4RS)-2-vinylbicyclo[2.2.1]heptan-2-ene (152 mmol) in pentane was added slowly dropwise. The solution was then allowed to slowly warm to ambient temperature then stirred for a further 12 hours and poured into ice/water. The aqueous phase was extracted with ether, washed with saturated ammonium chloride, brine, dried over sodium sulfate, filtered and the solvents removed in vacuo to yield the crude ketone. Further purification by bulb to bulb distillation 0.06 mbar at 150-155°C gave the desired crude ketone (purity: 90% and in the form of a mixture of stereoisomers wherein the four majour stereoisomers are in percentage ratio of 11:44:4:15, yield = 46%).

The crude ketone thus obtained was further purified by flash chromatography Puriflash cartridge (400g 50) with a gradient of cyclohexane:ethyl acetate, 499:1, 498:2, 497:3, 496:4, 495:5 (500 mL) etc as eluent, to provide a fraction of the ketone as a mixture of isomers and a fraction composed of the pure major isomer ketone (the one present in 44% in the crude ketone).

The major isomer (1-((1RS,4SR,6RS)-5,6-dimethyl-1,2,3,4,4a,5,6,7-octahydro-1,4-methanonaphthalen-6-yl)ethanone, the endo diastereomer of formula (II)) was isolated pure and had the following spectral data:
¹³C NMR: 213.8 (q), 149.4 (q), 109.8 (CH), 51.0 (q), 46.5, 44.8 (CH), 37.9 (CH₂), 37.3, 36.6 (CH), 35.3, 31.5, 25.9 (CH₂), 24.9, 16.5, 14.4 (CH₃)

### 1-(5,6-dimethyl-1,2,3,4,4a,5,6,7-octahydro-1,4-methanonaphthalen-6-yl)ethanol

A solution of 1-5,6-dimethyl-1,2,3,4,4a,5,6,7-octahydro-1,4-methanonaphthalen-6-yl)ethanone obtained above (crude ketone) (600 mg, 2.75 mmol) in diethyl ether (3 mL) was added to a stirred suspension of LiAlH₄ (52 mg, 1.38 mmol) in diethyl ether (7 mL) at ambient temperature. After 2 hours, the suspension was cooled in an ice bath and water (50 uL) then 15% aqueous NaOH (52 uL) then water 156 uL were added successively followed by anhydrous sodium sulfate (100 mg) and the white suspension stirred for a further 30 minutes at ambient temperature then filtered and the solvents removed in vacuo. Further purification by bulb to bulb distillation (Kugelrohr) 160°C at 0.1 mbar gave the alcohol (purity: 92% and in the form of a mixture of stereoisomers wherein the four majour stereoisomers are in percentage ratio of 10:47:10:3, yield: 75%).
Major isomer:
¹³C NMR: 148.7 (q), 111.7 (CH), 72.1 (CH), 47.4, 44.5 (CH), 39.3 (q) 38.0 (CH₂), 37.6, 35.3 (CH), 31.8, 31.6, 26.1 (CH₂), 18.1, 17.4, 12.9 (CH₃)

## Claims

1. A compound of formula 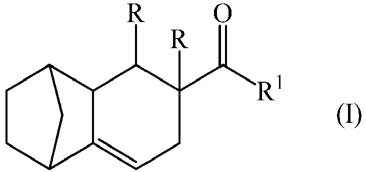 in the form of any one of its stereoisomers or a mixture thereof, and wherein each R, independently from each other, represents a hydrogen atom or a methyl or ethyl group provided that at least one of said R groups represents a methyl or ethyl group; and
R¹ represents a methyl or ethyl group.

2. A compound according to claim 1, **characterized in that** said compound is 1-((1RS,4SR,6RS)-5,6-dimethyl-1,2,3,4,4a,5,6,7-octahydro-1,4-methanonaphthalen-6-yl)ethanone.

3. Use as perfuming ingredient of a compound of formula (I) as defined in claim 1 or 2.

4. A perfuming composition comprising
i) at least one compound of formula (I), as defined in claim 1 or 2;
ii) at least one ingredient selected from the group consisting of a perfumery carrier and a perfumery base; and
iii) optionally at least one perfumery adjuvant.

5. A perfuming consumer product comprising-at least one compound of formula (I), as defined in claim 1 or 2.

6. A perfuming consumer product according to claim 5, **characterized in that** the perfumery consumer product is a perfume, a fabric care product, a body-care product, an air care product or a home care product.

7. A perfuming consumer product according to claim 5, **characterized in that** the perfumery consumer product is a fine perfume, a cologne, an after-shave lotion, a liquid or solid detergent, a fabric softener, a fabric refresher, an ironing water, a paper, a bleach, a shampoo, a coloring preparation, a hair spray, a vanishing cream, a deodorant or antiperspirant, a perfumed soap, shower or bath mousse, oil or gel, a hygiene product, an air freshener, a "ready to use" powdered air freshener, a wipe, a dish detergent or hard-surface detergent.

## Patentansprüche

1. Verbindung der Formel 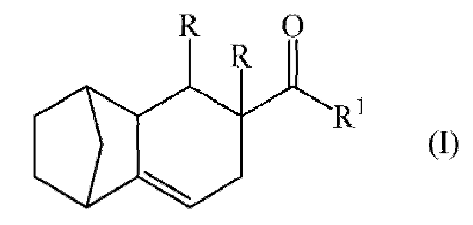 in der Form von einem ihrer Stereoisomere oder einem Gemisch davon, und wobei jedes R, unabhängig voneinander, ein Wasserstoffatom oder eine Methyl- oder Ethylgruppe darstellt, mit der Maßgabe, dass mindestens eine von den R-Gruppen eine Methyl- oder Ethylgruppe darstellt; und
R¹ eine Methyl- oder Ethylgruppe darstellt.

2. Verbindung nach Anspruch 1, **dadurch gekennzeichnet, dass** die Verbindung 1-((1RS,4SR,6RS)-5,6-Dimethyl-1,2,3,4,4a,5,6,7-octahydro-1,4-methanonaphthalin-6-yl)ethanon ist.

3. Verwendung als parfümierender Bestandteil einer Verbindung der Formel (I), wie in Anspruch 1 oder 2 definiert.

4. Parfümierende Zusammensetzung, umfassend
i) mindestens eine Verbindung der Formel (I), wie in Anspruch 1 oder 2 definiert;
ii) mindestens einen Bestandteil, ausgewählt aus der Gruppe, bestehend aus einem Träger für Parfümfabrikation und einer Basis für Parfümfabrikation; und
iii) wahlweise mindestens einen Hilfsstoff für Parfümfabrikation.

5. Parfümierendes Verbraucherprodukt, umfassend mindestens eine Verbindung der Formel (I), wie in Anspruch 1 oder 2 definiert.

6. Parfümierendes Verbraucherprodukt nach Anspruch 5, **dadurch gekennzeichnet, dass** das Verbraucherprodukt der Parfümfabrikation ein Parfüm, ein Gewebepflegeprodukt, ein Körperpflegeprodukt, ein Luftpflegeprodukt oder ein Hauspflegeprodukt ist.

7. Parfümierendes Verbraucherprodukt nach Anspruch 5, **dadurch gekennzeichnet, dass** das Verbraucherprodukt der Parfümfabrikation ein feines Parfüm, ein Kölnischwasser, eine Aftershavelotion, ein flüssiges oder festes Detergens, ein Weichspüler, ein Gewebeauffrischer, ein Bügelwasser, ein Papier, ein Bleichmittel, ein Shampoo, eine Farbzubereitung, ein Haarspray, eine Tagescreme, ein Deodorant oder Antiperspirant, eine parfümierte Seife, Dusch- oder Badeschaum, -öl oder -gel, ein Hygieneprodukt, ein Luftverbesserer, ein "gebrauchsfertiger" pulverförmiger Luftverbesserer, ein Wischtuch, ein Geschirrspülmittel oder Detergens für harte Oberflächen ist.

## Revendications

1. Composé de formule 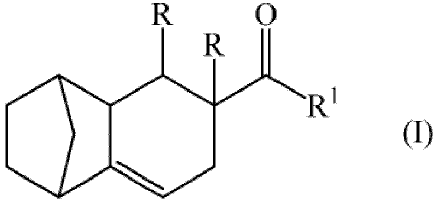 sous la forme de l'un quelconque de ses stéréoisomères ou d'un mélange de ceux-ci, et dans lequel chaque R, indépendamment des autres, représente un atome d'hydrogène ou un groupe méthyle ou éthyle sous réserve qu'au moins un desdits groupes R représente un groupe méthyle ou éthyle; et
R¹ représente un groupe méthyle ou éthyle.

2. Composé selon la revendication 1, **caractérisé en ce que** ledit composé est la 1-((1RS,4SR,6RS)-5,6-diméthyl-1,2,3,4,4a,5,6,7-octahydro-1,4-méthanonaphtalén-6-yl)-éthanone.

3. Utilisation en tant qu'ingrédient parfumant d'un composé de formule (I) tel que défini dans la revendication 1 ou 2.

4. Composition parfumante comprenant
i) au moins un composé de formule (I), tel que défini dans la revendication 1 ou 2;
ii) au moins un ingrédient choisi dans le groupe constitué d'un support de parfumerie et d'une base de parfumerie; et
iii) éventuellement, au moins un adjuvant d'usage courant en parfumerie.

5. Produit de consommation parfumant comprenant au moins un composé de formule (I), tel que défini dans la revendication 1 ou 2.

6. Produit de consommation parfumant selon la revendication 5, **caractérisé en ce que** le produit de consommation de parfumerie est un parfum, un produit d'entretien pour textile, un produit de soin corporel, un produit de traitement de l'air ou un produit d'entretien ménager.

7. Produit de consommation parfumant selon la revendication 5, **caractérisé en ce que** le produit de consommation de parfumerie est un parfum fin, une eau de Cologne, une lotion après-rasage, un détergent solide ou liquide, un adoucissant pour textile, une eau de linge, une eau de repassage, un papier, un agent de blanchiment, un shampooing, une préparation colorante, une laque capillaire, une crème de jour, un déodorant ou un anti-transpirant, un savon parfumé, une mousse, une huile ou un gel de bain ou de douche, un produit d'hygiène, un désodorisant d'air ambiant, un désodorisant d'air ambiant en poudre « prêt à l'emploi », une lingette, un détergent pour la vaisselle ou un détergent pour surfaces dures.
